# EUROPEAN PATENT APPLICATION

(11) **EP 4 538 287 A1**
(43) Date of publication of application: **16.04.2025**
(21) Application number: 23202750.8
(22) Date of filing: 10.10.2023
(51) Int. Cl.: C07K 16/12, A61K 39/09, A61P 31/04

(54) **NOVEL ENGINEERED ANTI-STREPTOCOCCUS ANTIBODIES**

(71) Applicant: Tanea Medical AB, 753 20 Uppsala (SE)
(72) Inventor: NORDENFELT, Bill Pontus, 753 20 Uppsala (SE); IZADI, Arman, 753 20 Uppsala (SE)
(74) Representative: Zacco Denmark A/S

(57) **Abstract**

The present invention generally relates to immunology and antibody engineering, and specifically to engineered antibodies which have improved properties. More specifically, the present invention provides an engineered antibody comprising an lgG3 subclass specific domain and a variable domain having a part binding to the M protein of group A streptococci.

## Description

### Technical field of the invention

The present invention relates to the fields of immunology, vaccines and pharmaceuticals products. Specifically the invention relates to antibodies, pharmaceutical compositions, antibody engineering, bacterial vaccines and methods for preparing and using same.

### Background of the invention

The immune system generates antibodies against invading pathogens as an essential part of the adaptive immune response. VDJ gene recombination in B cells defines the basic antigen-binding properties of antibodies (1,2), and B cells selected within the lymphoid tissues undergo class-switching and somatic hypermutation. The classical model of antibody variable and constant domain independence describes that classswitching only alters antibody effector function and does not alter affinity (3). However, some findings suggest that the constant domain could influence the binding to antigens (4-7), providing a potential mechanism where subclass and class-switching add further affinity diversity in addition to VDJ-recombination and somatic hypermutation (4-5). The human IgG antibody class contains four subclasses with varying abilities to trigger effector functions such as complement activation, phagocytosis, and cell cytotoxicity. These immune activities are mediated through binding to Fc-receptors on the surface of immune cells or binding to soluble complement proteins through the Fc-portion (1). These subclasses differ in the constant region of the heavy chain. Major differences between the subclasses lie in the hinge region that connects the Fab to the Fc domains. Although there are at least 13 allotypes of the IgG3 subclass (1), a common trait is that they have the most extended hinge region. This increased hinge length makes them more flexible, while the shorter hinge region of IgG2 is the most rigid (8). Furthermore, IgG3 is most efficient at activating the classical complement pathway (9). IgG3 and IgG1 have similar affinities to Fc-gamma receptors in humans, while IgG2 and IgG4 overall have a lower receptor affinity (10).

*S. pyogenes* has several virulence factors, of which the M protein is one of the most studied due to its importance for virulence (12). It is a cellwall-attached protein with a dimeric coiled-coil structure that extends out from the surface of the bacteria (12). The M protein has several immune evasive mechanisms, such as binding antibodies via their Fc-portion, which can inhibit Fc-mediated phagocytosis (13-14) or reducing phagocytosis through antibody-induced fibronectin binding (15). In addition, the M protein inhibits complement-mediated phagocytosis by sequestering C4BP and fibrinogen binding (16). These mechanisms are important for *S. pyogenes* virulence and survival in the host. M protein is a target for vaccine development against *S. pyogenes.* However, no vaccines have been approved for clinical use due to a lack of efficacy or because they promote the emergence of autoreactive antibodies (17). The generation of distinct mAbs against *S. pyogenes* could be a viable therapeutic alternative, and one IgG1 mAb (Ab25) against the M protein of *Streptococcus pyogenes* has been generated (11). Indeed, the IgG1 Ab25 mAb was protective when used as a prophylactic in a mouse subcutaneous infection (11). It has been observed that enriched binding of polyclonal IgG3 antibodies to M protein was linked to enhanced phagocytosis (18). However, the importance of subclass for effective function against *S. pyogenes* or other bacterial pathogens is not well understood.

There exist some biological limitations to IgG3's utility as a drug *in vivo.* Of the four subclasses, IgG3 has the shortest half-life due to lower affinity to the neonatal Fc-receptor. Through Fc-engineering, the affinity to the Fc-neonatal receptor can be increased without diminishment to Fc-effector functions.

There is a need for improved monoclonal antibodies for the treatment and prevention of infections with group A streptococci, such as infections from *Streptococcus pyogenes.* In particular there is a need for monoclonal antibodies and compositions containing such antibodies which are more efficient and which protects against group A streptococci.

### Summary of the invention

The object of the present invention is to overcome certain drawbacks in antibodies and their use in the treatment or prevention of infections from group A streptococci. This is achieved by the present inventors who have designed novel antibodies (monoclonal antibodies, mAbs) which are engineered forms of antibodies prepared by immunization using group A streptococci or the M protein thereof as antigen.

The present inventors found that subclass switching an IgG1 antibody binding to group A streptococci into an IgG3 antibody enhances opsonization and subsequent phagocytosis.

The present inventors further surprisingly found that engineering an IgG1 antibody binding to group A streptococci by substituting the IgG1 hinge with a sequence from an IgG3 hinge facilitated novel antibodies having improved properties. Hence, altering the constant domain of an antibody can strongly potentiate the immune response against group A streptococci, such as *Streptococcus pyogenes.*

Hence, antibody subclass engineering modulates the efficacy of antibodies as therapeutics against infections with group A streptococci.

Based on the foregoing findings, the present invention provides in a first aspect an engineered antibody comprising an IgG3 hinge and a variable domain having a part binding to the M protein of group A streptococci.

In one embodiment the engineered antibody comprises an IgG1 constant domain engineered with an IgG3 hinge.

The present invention further provides a composition comprising at least two different engineered antibodies according to the invention.

The present invention further provides a nucleic acid molecule encoding a protein comprising the engineered antibody as defined by the invention.

The present invention further provides a cell comprising the nucleic acid as defined by the invention.

The present invention further provides the use of the engineered antibody according to the invention for preventing, treating or alleviating an infection caused by group A streptococci.

The present invention further provides the use of the engineered antibody according to the invention for Fc-mediated phagocytosis of group A streptococci.

The present invention further provides the use of the composition according to the invention for activating the complement pathway.

The present invention further provides the use of the composition according to the invention for Fc-mediated phagocytosis of group A streptococci.

The present invention further provides

The following list of non-limiting items further illustrate the invention:
1. An engineered antibody comprising an IgG3 hinge and a variable domain having a part binding to group A streptococci.
2. The engineered antibody according to item 1, wherein the Fc region is an IgG3 region.
3. The engineered antibody according to item 2, wherein the Fc region comprises the IgG3 heavy chain constant domain SEQ ID NO:1 or a variant thereof having at least 90% identity, at least 95% identity, at least 98% identity or 100% identity to SEQ ID NO:1.
4. The engineered antibody according to item 2, wherein the IgG3 region comprises the three substitutions R435H, N392K and M397V.
5. The engineered antibody according to any of items 3-4, wherein said IgG3 region comprises a polypeptide having at least 95% identity or at least 98% identity to the modified IgG3 heavy chain constant domain of SEQ ID NO:2.
6. The engineered antibody according to any of items 3-5, wherein said IgG3 region comprises the modified IgG3 heavy chain constant domain of SEQ ID NO:2.
7. The engineered antibody according to item 1, wherein the Fc region is an IgG1 region.
8. The engineered antibody according to item 7, wherein said engineered antibody comprises an IgG1 constant domain engineered with an IgG3 hinge, e.g. SEQ ID NO:3.
9. The engineered antibody according to any of items 1-8, wherein said part binding to group A streptococci binds to the M protein.
10. The engineered antibody according to any of items 1-9, wherein said streptococci are *Streptococcus pyogenes.*
11. The engineered antibody according to any of items 1-10, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:4).
12. The engineered antibody according to any of items 1-10, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCP (SEQ ID NO:15).
13. The engineered antibody according to any of items 1-10, wherein said IgG3 hinge comprises an amino acid sequence which has at least 90% identity, at least 95% identity, at least 98% identity or 100% identity to the amino acid sequence X-(Y)n, wherein
   X is ELKTPLGDTTHTCPRCP (SEQ ID NO:5),
   Y is EPSKCDTPPPCPRCP (SEQ ID NO:6), and
   n is an integer in the range from 2 to 8, preferably in the range from 2 to 5, more preferably in the range from 2 to 4.
14. The engineered antibody according to any of items 1-13, which promotes opsonisation.
15. The engineered antibody according to any of items 1 and 7-14, wherein said engineered antibody has a CDR H3 loop selected from SEQ ID NO:7, SEQ ID NO:8, SEQ ID NO:9 and SEQ ID NO:10.
16. The engineered antibody according to any of items 1 and 7-15, wherein said engineered antibody has a CDR L3 loop selected from SEQ ID NO:11, SEQ ID NO:12, SEQ ID NO:13 and SEQ ID NO:14.
17. The engineered antibody according to any of items 1, 7-16 which has a CDR H3 loop and CDR L3 loop combination selected from SEQ ID NO:7 plus SEQ ID NO:11, SEQ ID NO:8 plus SEQ ID NO:12, SEQ ID NO:9 plus SEQ ID NO:13, and SEQ ID NO:10 plus SEQ ID NO:14.
18. The engineered antibody according to any of items 1, 7-17 which has a CDR H3 loop and CDR L3 loop combination where the CDR H3 loop is SEQ ID NO:7 and the CDR L3 loop is SEQ ID NO:11.
19. The engineered antibody according to any of the preceding items which has the six CDRs of Ab25, Ab26, Ab32 and Ab49 as described in WO22/058584.
20. The engineered antibody according to any of the preceding items which exhibit binding to at least one epitope of the M protein, such as binding to two different epitopes of the M protein.
21. The engineered antibody according to any of the preceding items for use in the treatment of an infection with group A streptococci.
22. A composition comprising at least two different engineered antibodies as defined in any of items 1-21.
23. The composition according to item 22, comprising from 3 to 15, from 3 to 12, from 3 to 9, from 5 to 15, from 5 to 10, or from 6 to 10 different engineered antibodies as defined in any of items 1-21.
24. A nucleic acid molecule encoding a protein comprising the engineered antibody as defined in any of items 1-21.
25. A cell comprising the nucleic acid as defined in item 24 such that the engineered antibody can be produced by said cell.
26. Use of the engineered antibody as defined in any of items 1-21 or the composition as defined in item 17, for preventing, treating or alleviating an infection caused by group A streptococci.
27. Use of the engineered antibody as defined in any of items 1-21 or the composition as defined in items 24-25, for Fc-mediated phagocytosis of group A streptococci.
28. Use of the engineered antibody as defined in any of items 1-21 or the composition as defined in any of items 22-23, for activating the complement pathway.
29. A method for preparing an engineered antibody as defined in any of items 1-21, comprising the steps:
   - immunization of a host organism with said group A streptococci or an M protein therefrom,
   - isolation of at least one group A streptococci reactive B-cell to isolate an IgG1 antibody binding to said group A streptococci,
   - preparing at least one recombinant DNA molecule which encodes a modified version of said IgG1 antibody comprising the replacing of the IgG1 subclass specific domain of said isolated IgG1 antibody with the IgG3 subclass specific domain,
   - expressing said at least one recombinant DNA molecule in a suitable host cell,
   to obtain said engineered antibody.
30. A method for preparing an engineered antibody as defined in any of items 1-21, comprising the steps:
   - immunization of a host organism with said group A streptococci or the M protein therefrom,
   - isolation of at least one group A streptococci reactive B-cell to isolate an IgG1 antibody binding to said group A streptococci,
   - preparing at least one recombinant DNA molecule which encodes a modified version of said IgG1 antibody, said modification comprising the replacing of the IgG1 hinge of said isolated IgG1 antibody with an IgG3 hinge,
   - expressing said at least one recombinant DNA molecule in a suitable host cell,
to obtain said engineered antibody.

### Brief description of the figures

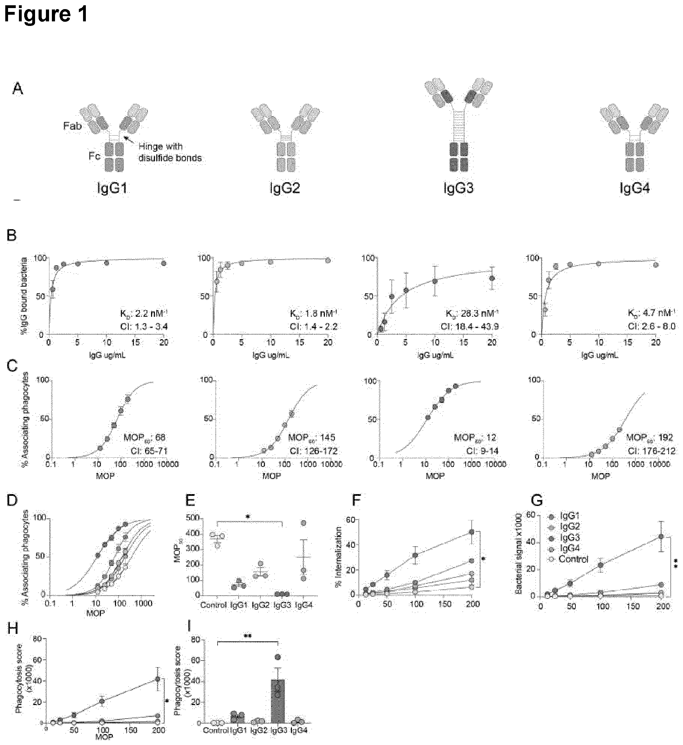

**Figure 1****.** IgG3 exhibits potent phagocytosis efficacy despite reduced binding to antigen. **A** The four subclasses of Ab25. The antibodies consist of two identical heavy chains and identical light chains. The variable heavy domain, the constant domain of the light chain, and the variable light domain are identical for the four antibodies. The constant domains of the heavy chains are depicted for IgG1, IgG2, IgG3, and IgG4. **B** Affinity curves for the IgG subclasses. The Y-axis is the percentage of GFP-expressing bacteria positive for antibodies. The KD values (nM-1) with a 95% confidence interval are shown in each respective graph. Data points are from three independent experiments. **C** MOP50 curves of the subclasses, bacteria were opsonized with 15 ug/mL of each respective antibody or control. The Y-axis shows the percentage of THP-1 cells that are positive for bacteria, while the X-axis depicts the ratio of bacteria to phagocytes added (MOP). The MOP50 values with a 95% confidence interval are shown in each graph. The data points are from three independent experiments. **D** Shows the MOP50 curves of all the antibody treatments in one graph. Control is Xolair, human IgG1 anti-IgE. E Individual MOP50 values from the three independent experiments. **F** Percentage of THP-1 cells with internalized bacteria across the MOP range. **G** Amount of bacteria that are phagocytosed by the whole THP-1 cell population measured as median MFI of FITC-A (bacterial signal, Oregon Green). **H-I** Phagocytosis score for each antibody treatment is shown with MOP on the X-axis for H, while in I, the MOP is 200. All statistical analysis was done, comparing the treatments to the control antibody with Kruskal-Wallis multiple comparisons corrected by Dunn's correction test. * denotes P-value < 0.05 and P-value > 0.05 is ns. The data points in figure B-I represent the mean value, and the error bars are in SEM.

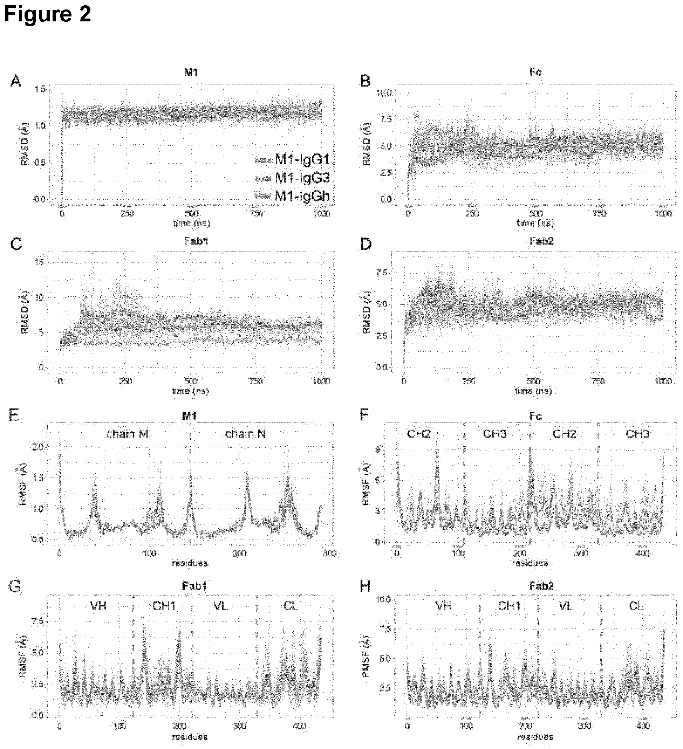

**Figure 2****.** The root mean square deviations and fluctuations for M1-lgGs. The RMSD from the equilibrated structure was computed on the C& atoms of each replicate and every domain: a) M1, b) Fc, c) Fab1, and d) Fab2. The RMSF was measured on the C& atoms with respect to the average conformation and averaged by residue, considering the last 900 ns of the MD simulations for the e) M1, f) Fc, g) Fab1, and h) Fab2 domains. The average values over the three replicates of each domain are reported for the M1-IgG1, M1-IgG3, and M1- IgGh systems, respectively. The shades correspond to the standard deviations.

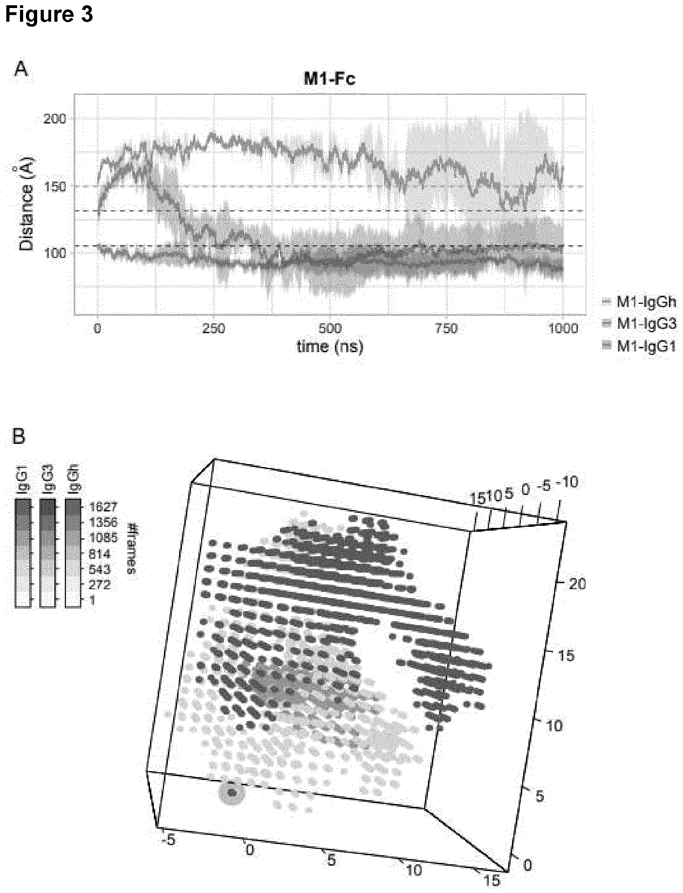

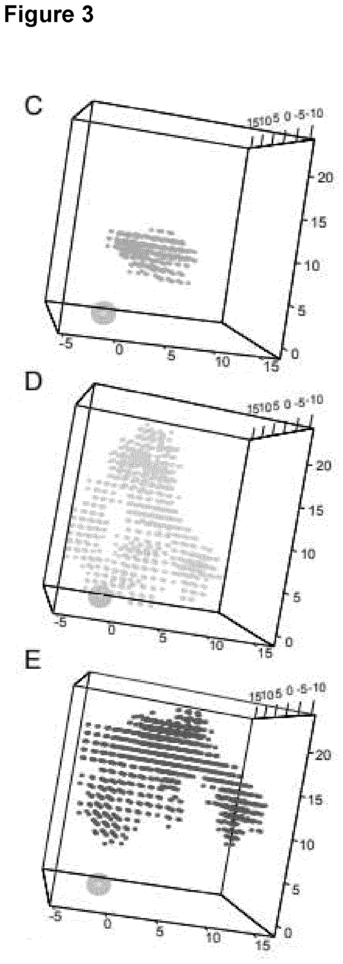

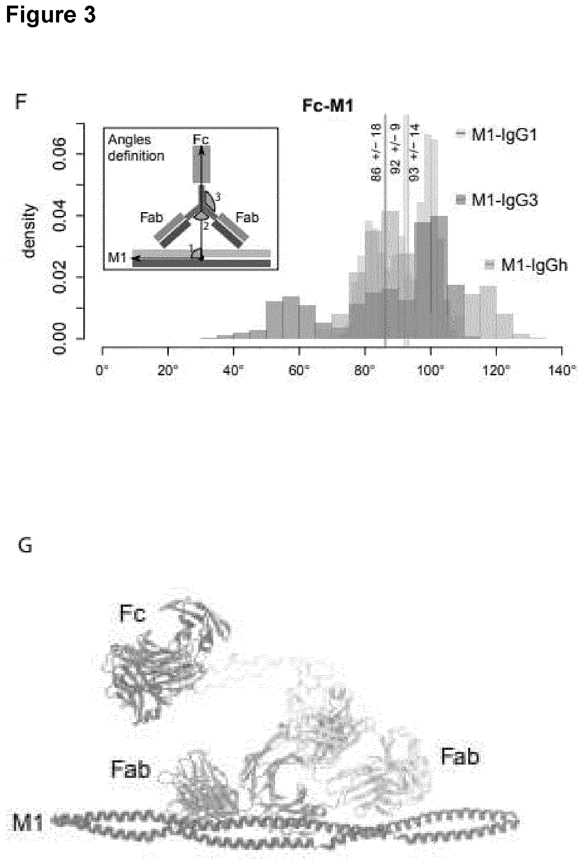

**Figure 3****.** The IgG3 hinge provides broad Fc spatial mobility spectra and flexibility relative to M protein. **A** The average distance between the center of mass of the M1 protein and the Fc domain of the IgG, IgG3, and IgGh was measured over the three replicates of each system (reported in pink and cyan, respectively). The shades correspond to standard deviations, and the initial distances are shown with dashed lines. **B** The center of mass of the Fc domain of IgG1 (in blue), IgG3 (in red), and IgGh (green) at every snapshot of the simulations are depicted. The intensity of the colors represents the population of each point. The center of mass of M1 is shown with a gray circle at the origin. The individual distribution of points are reported for **C** IgG1-M1, **D** IgG3-M1, and **E** IgGh-M1 simulations. The angle changes formed between the Fc domain and protein M1 are reported for IgG1, IgG3, and IgGh when both Fabs contact the protein M1. The average values are shown with lines. The vectors forming the angle are depicted in the inset. **G** The most representative conformation of the M1-IgG3 is reported when dual-Fab binding was observed. The IgG-M1 models in the figure are the starting models for the MD simulation

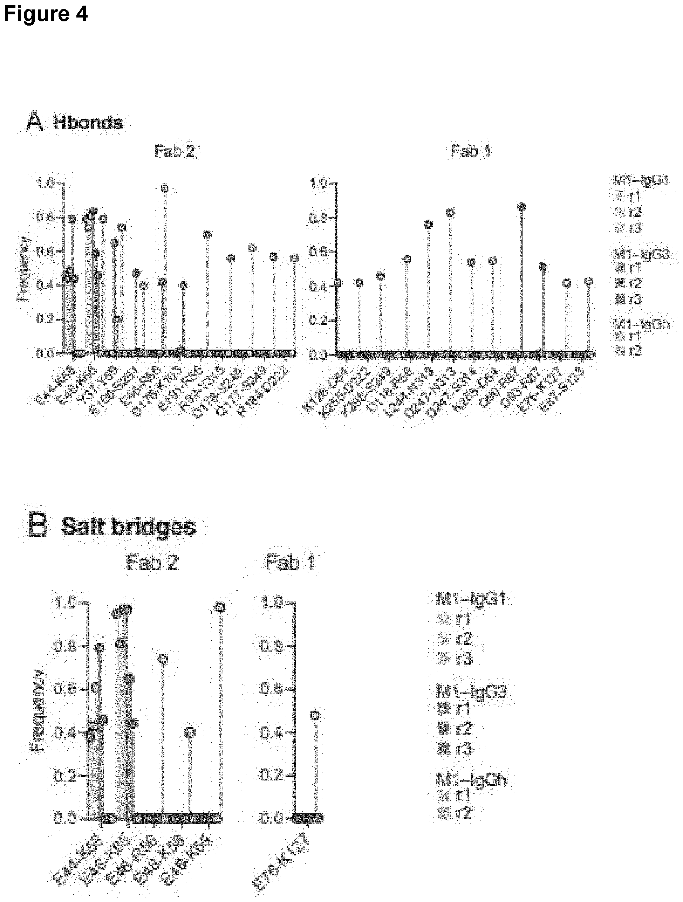

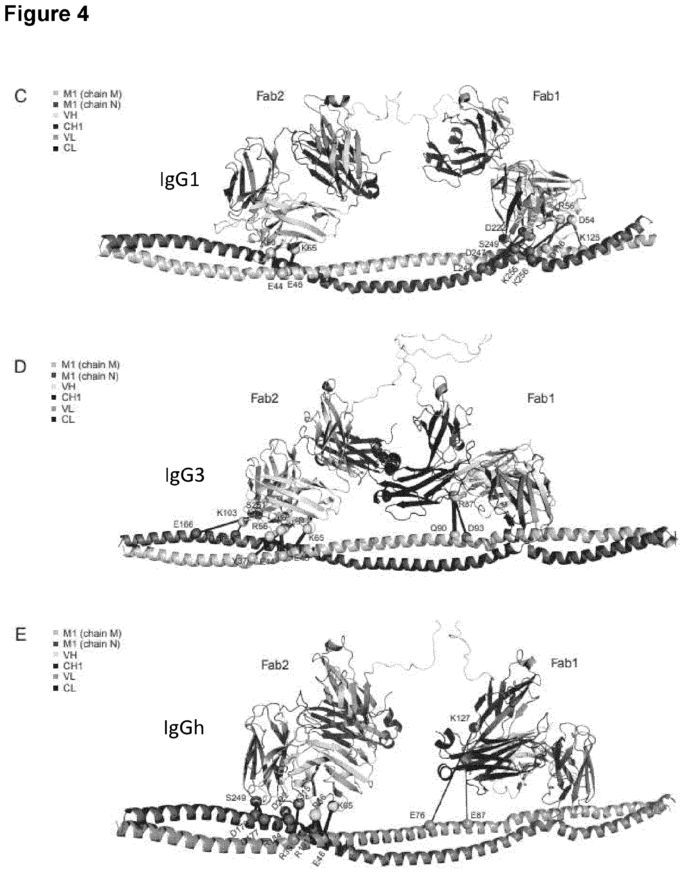

**Figure 4****.** The constant domain influences the interaction network between the IgG Fabs and M1 at an atomic level. The interaction network at the interface of Fab domain and protein M1. The frequency of **A** H-bonds and **B** salt bridges formed in every replicate of the IgG1-M1 and IgG3-M1 simulations. The interactions are also shown on the structure of **C** IgG1-M1, **D** IgG3-M1, and **E** IgGh-M1. The residues involved in the interactions are shown as spheres. The two chains of protein M1 are colored in light and dark purple, and the VH, CH1, VL, and CL domains are shown in light green, dark green, pink, and red, respectively. The IgG-M1 models in the figure are the starting models for the MD simulations.

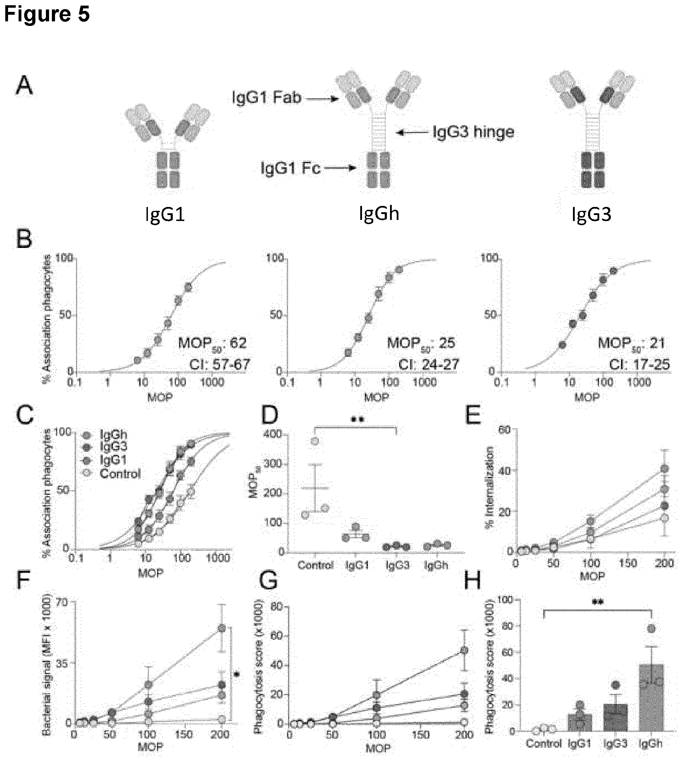

**Figure 5****.** IgGh retains the high affinity of IgG1 and gains superior opsonic function. **A** Schematic of IgG1, IgG3, and the IgGh The antibodies consist of two identical heavy chains and identical light chains. The variable heavy domain, the constant domain of the light chain, and the variable light domain are identical for the three antibodies. The constant domains of the heavy chains are depicted in different colors: blue for IgG1 and red for IgG3. **B** MOP50 curves of the subclasses, bacteria were opsonized with 15 ug/mL of each respective antibody. The Y-axis shows the percentage of THP-1 cells that are positive for bacteria, while the Xaxis depicts the ratio of bacteria to phagocytes added (MOP). The MOP50 values with a 95% confidence interval are shown in each graph. The data points are from three independent experiments. **C** Shows the MOP50 curves of all the antibody treatments in one graph. Control is Xolair, human IgG1 anti-IgE. D Individual MOP50 values from the three independent experiments. **E** Percentage of THP-1 cells with internalized bacteria across the MOP range. **F** Amount of bacteria that are phagocytosed by the whole THP1 cell population measured as median MFI of FITC-A (bacterial signal, Oregon Green). **G-H** Phagocytosis score for each antibody treatment is shown with MOP on the X-axis for G, while in H, the MOP is 200. All statistical analysis was done, comparing the treatments to the control antibody with Kruskal-Wallis multiple comparisons corrected by Dunn's correction test. * denotes P-value < 0.05 and P-value > 0.05 is ns. The data points in figure B-I represent the mean value, and the error bars are in SEM.

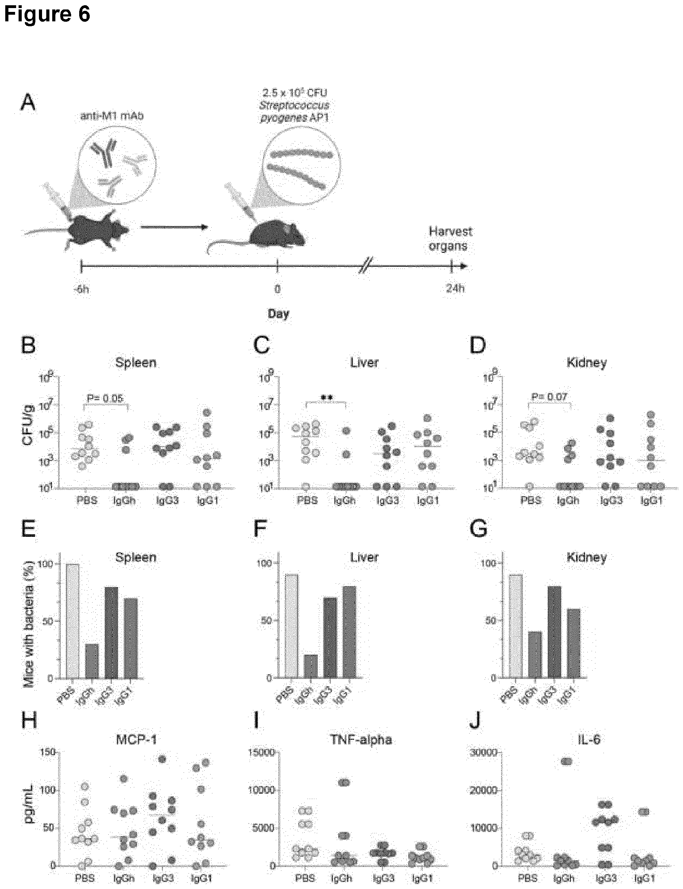

**Figure 6****.** Increased hinge flexibility of IgG1 confers antibody protection against systemic infection in mice. **A** Schematic of the animal experiment: pre-treatment 0.4 mg of antibody treatment or PBS 6 hours before infection with AP1. Animals were sacrificed 24 hours post-infection, and organs and blood were harvested for analysis. **B-D** Shows pooled data from two independent experiments of 5 mice in each cohort. Bacterial load (CFU/ g) in each organ was determined by serial dilution and viable count determination after overnight incubation. **E-G** shows the percentage of mice positive for bacteria in each respective organ for all treatments. **H-J** Cytokine levels (pg/ml) in plasma were measured using a cytometric bead array. In **B-C,** the median is shown. Statistical analysis was performed, comparing the treatments to the PBS, by Kruskal-Wallis with multiple comparisons and Dunn's correction test. ** denotes P-value < 0.01, * denotes P< 0.05 and P-value > 0.05 is ns.

### Detailed description of the invention

Unless specifically defined herein, all technical and scientific terms used have the same meaning as commonly understood by a skilled artisan in the fields of immunology, biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory). Kuby Immunology (2018, J Punt & S Stranford). Therapeutic Antibody Engineering (2012, 1st Ed), W Strohl & L Strohl.

The present invention provides an engineered antibody comprising an IgG3 hinge and a variable domain having a part binding to the M protein of group A streptococci.

In an embodiment the Fc region is an IgG3 region.

In another embodiment the Fc region comprises the IgG3 heavy chain constant domain SEQ ID NO:1 or a variant thereof having at least 90% identity, at least 95% identity, at least 98% identity or 100% identity to SEQ ID NO:1. In yet another embodiment the IgG3 region comprises the three substitutions R435H, N392K and M397V. In another embodiment the IgG3 region comprises a polypeptide having at least 95% identity or at least 98% identity to the modified IgG3 heavy chain constant domain of SEQ ID NO:2. In another embodiment the IgG3 region comprises the modified IgG3 heavy chain constant domain of SEQ ID NO:2.

In a preferred embodiment the Fc region is an IgG1 region.

In another embodiment the engineered antibody comprises an IgG1 constant domain engineered with an IgG3 hinge, e.g. SEQ ID NO:3.

In another embodiment the part binding to group A streptococci binds to the M protein.

In another embodiment the group A streptococci are *Streptococcus pyogenes.*

In another embodiment the IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:4).

In another embodiment the IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCP (SEQ ID NO:15).

In another embodiment the IgG3 hinge comprises an amino acid sequence which has at least 90% identity, at least 95% identity, at least 98% identity or 100% identity to the amino acid sequence X-(Y)n, wherein
X is ELKTPLGDTTHTCPRCP (SEQ ID NO:5),
Y is EPSKCDTPPPCPRCP (SEQ ID NO:6), and
n is an integer in the range from 2 to 8, preferably in the range from 2 to 5, more preferably in the range from 2 to 4.

In another embodiment the engineered antibody promotes opsonisation.

The antibody according to any the present invention preferably binds to streptococcal M protein with a K_{D} of less than 5 × 10⁻⁸ M⁻¹, as determined by binding to *Streptococcus pyogenes* SF370. In one embodiment the antibody binds to streptococcal M protein with a K_{D} less than 50 × 10⁻⁹ M⁻¹, less than 15 × 10⁻⁹ M⁻¹ or less than 1 × 10⁻⁹ M⁻¹.

In a further embodiment the antibody of the invention has said CDR H3 loop selected from SEQ ID NO:7, SEQ ID NO:8, SEQ ID NO:9 and SEQ ID NO:10.

SEQ ID NO: 7 is CARSYPHKRWLRPPFDYW.

SEQ ID NO: 8 is CAKNSRSGWYFFFDYWGQ.

SEQ ID NO: 9 is CARQGFDTRGEDAFEIWG.

SEQ ID NO: 10 is CVRDSRFWGIFDYWGQGT.

In a further embodiment the engineered antibody has a CDR L3 loop selected from SEQ ID NO:11, SEQ ID NO:12, SEQ ID NO:13 and SEQ ID NO:14.

SEQ ID NO: 11 is QYNSYPVTFGQGTKV

SEQ ID NO: 12 is QYDNLPLTFGGGTKV

SEQ ID NO: 13 is QRSGWPSIFTFGPGTKV

SEQ ID NO: 14 is QRSNWPPTFGQGTKV

In a further embodiment the engineered antibody has a CDR H3 loop and CDR L3 loop combination selected from SEQ ID NO:7 plus SEQ ID NO:11, SEQ ID NO:8 plus SEQ ID NO:12, SEQ ID NO:9 plus SEQ ID NO:13, and SEQ ID NO:10 plus SEQ ID NO:14.

In a further embodiment the engineered antibody has the six CDRs of Ab25, Ab26, Ab32 and Ab49 as described in WO22/058584.

In another embodiment the engineered antibody exhibits binding to at least one epitope of the M protein, such as binding to two different epitopes of the M protein.

In another embodiment the engineered antibody is for use in the treatment of an infection with group A streptococci.

Compositions and formulations in accordance with the present invention are prepared in accordance with known standards for the preparation of pharmaceutical compositions and formulations. For instance, the compositions and formulations are prepared in a way that they can be stored and administered appropriately, e.g. by using pharmaceutically acceptable components such as carriers, excipients or stabilizers. Such pharmaceutically acceptable components are not toxic in the amounts used when administering the pharmaceutical composition or formulation to a patient. The pharmaceutical acceptable components added to the pharmaceutical compositions or formulations may depend on the chemical nature of the inhibitor and targeting agent present in the composition or formulation (depend on whether the targeting agent is e.g. an antibody or fragment thereof or a cell expressing a chimeric antigen receptor), the particular intended use of the pharmaceutical compositions and the route of administration.

In a preferred embodiment in accordance with the invention, the composition or formulation is suitable for administration to humans, preferably the formulation is sterile and/or non-pyrogenic.

### Definition of some terms.

Unless otherwise defined below, the terms used in the present invention shall be understood in accordance with the common meaning known to the person skilled in the art.

The term "antibody" (e.g. a monoclonal antibody) as described herein shall be understood in accordance with the common meaning known to the person skilled in the art. The antibody may be a derivatized antibody or be linked to a different molecule. For example, molecules that may be linked to the antibody are other proteins (e.g. other antibodies), a molecular label (e.g. a fluorescent, luminescent, colored or radioactive molecule), a pharmaceutical and/or a toxic agent. The antibody or antigen-binding portion may be linked directly (e.g. in form of a fusion between two proteins), or via a linker molecule (e.g. any suitable type of chemical linker known in the art).

The term "isolated antibody" as described herein is to be understood as an antibody which is recovered or purified to some extent. In an embodiment that extent means it is free from a multitude of antibodies having a different structure such as more than 25 antibodies. In another embodiment that extent means it is free from different antibodies not binding to the spike protein.

The term "engineered antibody" as described herein shall be understood in accordance with the common meaning known to the person skilled in the art. Engineered antibodies are novel recombinant antibody molecules with improved antigen specificities and effector functions, which are typically produced by the recombinant DNA and protein engineering technologies.

As used herein, the term "IgG3 hinge" shall be understood to mean the hinge region from the IgG3 class of antibodies, including variants thereof. Non-limiting examples of IgG3 hinges are peptides having an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:4). Other examples of IgG3 hinges have an amino acid sequence which is at least 90% identity, at least 95% identity, at least 98% identity or 100% identity to the amino acid sequence X-(Y)n, wherein X is ELKTPLGDTTHTCPRCP (SEQ ID NO:5), Y is EPSKCDTPPPCPRCP (SEQ ID NO:6), and n is an integer in the range from 2 to 8, preferably in the range from 2 to 5, more preferably in the range from 2 to 4.

The term "bind" or "binding" in relation to antibodies as used herein refers to the forming of a complex with a molecule that is to be bound, e.g. theM protein of *Streptococcus pyogenes.* Binding typically occurs non-covalently by intermolecular forces, such as ionic bonds, hydrogen bonds and Van der Waals forces and is typically reversible. Various methods and assays to determine binding capability are known in the art. Binding is usually a binding with high affinity, wherein the affinity as measured in KD values is preferably less than 1 mM, more preferably less than 100 nM, even more preferably less than 10 nM, even more preferably less than 1 nM, even more preferably less than 100 pM, even more preferably less than 10 pM, and even more preferably less than 1 pM.

As used herein, each occurrence of terms such as "comprising" or "comprises" may optionally be substituted with "consisting of" or "consists of'.

Terms such as "treating", "preventing" or "alleviating" according to the present invention refer to a therapeutic treatment. An assessment of whether or not a therapeutic treatment works can, for instance, be made by assessing whether the treatment prevents or inhibits bacterial infection in the treated patient or patients. Preferably, the prevention or inhibition is statistically significant as assessed by appropriate statistical tests which are known in the art. Inhibition or prevention of bacterial infection may be assessed by comparing the degree of bacterial infection in a group of subjects infected or exposed to the virus which are administered an engineered antibody according to the present invention to a control group of subjects that are infected or exposed but who is administered placebo. Alternatively, it may be assessed by comparing a group of infected patients that receive a standard treatment of the art plus a treatment according to the invention with a control group of patients that only receive a standard treatment of the art. Such studies for assessing the inhibition or prevention of group A streptococcal infection are designed in accordance with accepted standards for clinical studies, e.g. double-blinded, randomized studies with sufficient statistical power.

As used herein, the term "pharmaceutically acceptable" means being approved by a regulatory agency of the Federal or a state government or listed in the U.S. Pharmacopia, European Pharmacopia or other generally recognized pharmacopia for use in mammals, and more particularly in humans. Pharmaceutically acceptable carriers include, but are not limited to, saline, buffered saline, dextrose, water, glycerol, sterile isotonic aqueous buffer, and combinations thereof.

The sequences referred to in the present description are the following :
SEQ ID NO:1 (lgG3 Heavy chain constant domain) 
ASTKGPSVFPLAPCSRSTSGGTAALGCLVKDYFPEPVTVSWNSGALTSGVHTFPAVLQSSGLYSLSSVVTVPSSSL
GTQTYTCNVNHKPSNTKVDKRVELKTPLGDTTHTCPRCPEPKSCDTPPPCPRCPEPKSCDTPPPCPRCPEPKSCD
TPPPCPRCPAPELLGGPSVFLFPPKPKDTLMISRTPEVTCVVVDVSHEDPEVQFKWYVDGVEVHNAKTKPREEQ
FNSTFRVVSVLTVLHQDWLNGKEYKCKVSNKALPAPIEKTISKTKGQPREPQVYTLPPSREEMTKNQVSLTCLVK
GFYPSDIAVEWESSGQPENNYNTTPPMLDSDGSFFLYSKLTVDKSRWQQGNIFSCSVMHEALHNRFTQKSLSLS
PGK
SEQ ID NO:2 (Modified IgG3 Heavy chain constant domain) 
ASTKGPSVFPLAPCSRSTSGGTAALGCLVKDYFPEPVTVSWNSGALTSGVHTFPAVLQSSGLYSLSSVVTVPSSSL
GTQTYTCNVNHKPSNTKVDKRVELKTPLGDTTHTCPRCPEPKSCDTPPPCPRCPEPKSCDTPPPCPRCPEPKSCD
TPPPCPRCPAPELLGGPSVFLFPPKPKDTLMISRTPEVTCVVVDVSHEDPEVQFKWYVDGVEVHNAKTKPREEQ
FNSTFRVVSVLTVLHQDWLNGKEYKCKVSNKALPAPIEKTISKTKGQPREPQVYTLPPSREEMTKNQVSLTCLVK
GFYPSDIAVEWESSGQPENNYKTTPPVLDSDGSFFLYSKLTVDKSRWQQGNIFSCSVMHEALHNHFTQKSLSLS
PGK
SEQ ID NO:3 (IgG1 constant domain engineered with IgG3 hinge) 
ASTKGPSVFPLAPSSKSTSGGTAALGCLVKDYFPEPVTVSWNSGALTSGVHTFPAVLQSSGLYSLSSVVTVPSSSL
GTQTYICNVNHKPSNTKVDKKVELKTPLGDTTHTCPRCPEPKSCDTPPPCPRCPEPKSCDTPPPCPRCPAPELLG
GPSVFLFPPKPKDTLMISRTPEVTCVVVDVSHEDPEVKFNWYVDGVEVHNAKTKPREEQYNSTYRVVSVLTVLH
QDWLNGKEYKCKVSNKALPAPIEKTISKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALHNHYTQKSLSLSPGK
SEQ ID NO:4 (lgG3 hinge region)
ELKTPLGDTTHTCPRCPEPKSCDTPPPCPRCPEPKSCDTPPPCPRCPEPKSCDTPPPCPRCP
SEQ ID NO:5 (lgG3 hinge part): ELKTPLGDTTHTCPRCP
SEQ ID NO:6 (lgG3 hinge part): EPSKCDTPPPCPRCP
SEQ ID NO: 7 (CDR H3 loop for Ab25): CARSYPHKRWLRPPFDYW
SEQ ID NO: 8 (CDR H3 loop for Ab26): CAKNSRSGWYFFFDYWGQ
SEQ ID NO: 9 (CDR H3 loop for Ab32): CARQGFDTRGEDAFEIWG
SEQ ID NO: 10 (CDR H3 loop for Ab49): CVRDSRFWGIFDYWGQGT
SEQ ID NO: 11 (CDR L3 loop for Ab25): QYNSYPVTFGQGTKV
SEQ ID NO: 12 (CDR L3 loop for Ab26): QYDNLPLTFGGGTKV
SEQ ID NO: 13 (CDR L3 loop for Ab32): QRSGWPSIFTFGPGTKV
SEQ ID NO: 14 (CDR L3 loop for Ab49): QRSNWPPTFGQGTKV
SEQ ID NO: 15 (IgG3 hinge region 47aa):
   ELKTPLGDTTHTCPRCPEPSKCDTPPPCPRCPEPSKCDTPPPCPRCP

### Examples

### Materials and Methods

### Plasmid generation, transformation and plasmid purification

To generate heavy chain plasmids of the different subclasses, pVITRO1-TrastuzumabIgG2 (Addgene plasmid #61884; RRID: Addgene_61884), IgG3 (Addgene plasmid #61885; RRID:Addgene_61885), and IgG4 (Addgene plasmid #61887; RRID:Addgene_61887) was a gift from Andrew Beavils lab (44). The heavy chain and light chain of Ab25 were generated as described previously in Bahnan et al (11). The cloning was done by amplifying the constant region of the heavy chains for IgG2, 3, and 4 by using the high-fidelity Phusion (NEB Biolabs) 3-step PCR protocol. The original heavy chain of Ab25 was linearized so that the constant region of IgG1 was removed. The primer design was planned so the amplified vector and insert contain overlapping ends, which are compatible with HIFI DNA assembly (NEB Biolabs). For the generation of the IgG1/3 hybrid, a 2-step PCR was done to amplify the insert and the vector, and the fragments were combined by HIFI DNA assembly. The plasmids were transformed into competent E. coli supplied by the manufacturer of the HIFI-DNA assembly according to their instructions. Following transformation, single colonies were picked and grown for 16 h 3 mL Luria Bertani (LB) medium containing 10 µg/mL ampicillin in a shaking incubator at 37 °C. The following day the bacteria culture was harvested and used for miniprep (QIAprep Spin Miniprep Kit, QIAGEN) to acquire pure plasmids. The plasmid concentration was measured by drop spectrometry (DeNovix) and was sent for sequencing to verify that no mutations had been introduced. For midi-preps, LB was inoculated with a single colony in 3 mL LB containing 10 µg/mL ampicillin and grown for 6 h in a shaking incubator at 37 °C. They were added to 27 mL LB containing 10 µg/mL ampicillin for 16 h under the same conditions. The culture was harvested the day after, and the plasmids were purified by QIAGEN plasmid plus midi kit according to the manufacturer's instructions.

### Bacteria culturing

*S. pyogenes* SF370 (emm1 type) was streaked on a plate with Todd-Hewitt Yeast (THY) agar with erythromycin (1:1000 dilution, 100 mg/mL) when the strain containing the GFP plasmid was grown. When the WT SF370 strain was grown, this was without erythromycin. The plate was kept in use for up to 2 weeks. The day before an experiment, one colony was added to 10 mL of THY media with erythromycin for the GFP expressing SF370 and without for the WT. After 16 hours, the overnight was diluted 1:20 and grown to mid-log for 2.5 h for the GFP and 2hr for the wild type. For the phagocytosis assay, the bacteria were heat-killed. After growing to mid-log, the cultures were washed once with PBS, put on ice for 15 minutes, put in a shaking heat block (300 rpm) at 80°C for 5 minutes, and then directly put back on ice for 15 minutes. The heat-killed bacteria were stained 1:500 with Oregon Green 488-X succinimidyl ester (2 µL of dye from stock in 1 mL of PBS) (Thermo Fisher) fluorescent dye for 30 minutes at 37 °C. After staining, the bacteria were washed with PBS, resuspended with sodium carbonate buffer (0.1M pH 8.3), and stained with CypHer5E (Fisher Scientific) 1:100 in a volume of 200 µL for 30 minutes at 37 °C, protected from light. The bacteria were washed twice with PBS and then resuspended in sodium medium (5.6 mM glucose, 127 mM NaCl, 10.8 mM KCI, 2.4 mM KH2PO4, 1.6 mM MgSO4, 10 mM HEPES, 1.8 mM CaCl2; pH adjusted to 7.3 with NaOH) and stored at 4 °C.

### Cell culture, antibody production, and antibody purification

THP-1 monocytic cells were cultured in RPMI with 10% FBS and L-glutamine and were kept at 5-10×105 cells per mL. The cells were never used more than 6 weeks after thawing the vials. 6.5 million human embryonic kidney (HEK) 293 cells (Gibco) were seeded the day before transfection in 150mm dishes in DMEM (Gibco) supplemented with 10% FBS (Gibco) and L-glutamine. Transfection was done by first treating the cells with chloroquine diluted 1:1000 in fresh DMEM to replace the old. 6 h after Chloroquine treatment, 20 µg of Heavy chain and Light chain plasmid was diluted in 1mL OptiMEM(Gibco) and 120 µL polyethyleneimine (1 mg/mL PEI, Thermo fisher) for each plate. The mix was left to incubate for 20 minutes at room temperature before gently being added to the plates. The following day the DMEM was replaced by OptiMEM with 2 wash steps with sterile PBS before the switch. The cells were left to incubate for 48 h, and the supernatants were collected and spun for 5 minutes at 450 x g to remove any cells. Expi293F suspension cells were purchased from Gibco (Thermo Fisher) and routinely cultured in 125 mL Erlenmeyer flasks (Nalgene) in 30 mL Expi293 medium (Gibco) in an Eppendorf s41i shaker incubator at 37°C, 8% CO2, 120 rpm. Cells were passaged and split to a density of 0.5 × 106 cells/mL every 3 to 4 days. The day before transfection, the cells were seeded at a density of 2 × 106 cells/mL. The next day, cells were seeded at 7.5 × 107 cells in 25.5 mL Expi293 medium. 20 µg of respective heavy and light chain plasmids were used to transfect Expi293 cells. Expi293 cells were transfected at a concentration of 3 million/mL in a volume of 25.5mL in Expi293 media. The plasmids were mixed with 2.9 mL of OptiMEM media and 100 µL of Expifectamine. The mix was left to incubate at room temperature for 15 min. After incubation, the mix was added to the cell flasks containing the cells. 22 hours later, 1.5 mL of enhancer 1 and 0.15 mL of enhancer 2 (Expifectamine kit, Gibco) were added to the cells. 72 hours later, the cell's supernatant was harvested. The supernatants (from both methods) were later purified the same day by incubation for 2 h with protein G sepharose (Cytiva). After incubation, the beads were washed 2 times, 50 mL PBS in a chromatography column (Biorad). Elution was done by adding 2x5 mL of HCl-glycine (0.1M, pH 2.5), and 1000 mL of TRIS (pH 8, 1M) was used to neutralize the pH. The purified antibodies were concentrated, and the buffer was exchanged to PBS by means of spin columns. The antibody concentrations were measured by doing an SDS-PAGE with serial dilutions of Xolair and Denovix drop spectroscopy using the IgG setting.

### Binding assays with secondary conjugated antibodies

For binding assays, live S.pyogenes strain SF370 transformed with GFP was used. 10mL of mid-log culture was concentrated and resuspended in a final volume of 1mL in PBS. The bacteria were sonicated for 2x2 minutes to break clumps. 10 µL of concentrated bacteria were added to each well of a 96-well low-binding plate containing 90 µL of anti-M antibodies (concentrations of antibodies varying between 20-0.65 µg/mL) in PBS. Serial dilutions of antibodies were prepared with concentrations ranging from 20 µg/mL to 0.65 µg/mL. The bacteria were opsonized for 30 min at 37 °C with gentle shaking. After opsonization, the bacteria were washed twice with PBS before adding 50 µL (1:500 dilution from stock) secondary antibody, Alexa Fluor^{®} 647 AffiniPure F(ab')₂ Fragment Goat Anti-Human IgG, F(ab')₂ fragment specific, (109-606-097,Jackson Immunoresearch). The secondary antibody was incubated for 30 minutes at 37 °C with gentle shaking. Following this, PBS was added so that the final volume reached 250 µL. The plates were analyzed by using a Cytoflex flow cytometer (Beckman Coulter). The data acquired from the flow cytometer experiments described above were processed on Flowjo software for gating strategy. The positive gate for the live GFP-expressing bacteria was set by gating for size (SSC) and GFP fluorescence (FITC) (Supp Fig. 1A). The gate for antibody reactivity to M was set by using a low concentration of Xolair (0.5 micrograms per mL) for Fcbackground binding (Supp. Fig. 1A). Antibody reactivity was determined by gating on percentage bound bacteria positive for the secondary antibody (APC) (Supp. Fig. 1A). The data were analyzed in GraphPad Prism, and the percentage of parents was plotted against antibody concentration followed by a non-linear regression analysis with Bmax constrained to 100% and KD unconstrained.

### Directly conjugated antibody binding assay

The antibodies were conjugated with Alexa Flour 647 using the GlyClick kit (Genovis) according to the manufacturer's protocol. The binding assay was performed with live SF370 expressing GFP. 10 mL of mid-log of culture was spun down and taken up in 1 mL PBS (10X concentration). The bacteria were sonicated as described previously. 10 µL of the bacteria from the 10x stock was added to 90 µL of antibodies in PBS. The concentrations of antibodies varied from 10-0.6 µg/mL for IgG1 and the hybrid. The bacteria were opsonized for 30 min at 37 °C with gentle shaking. 150 µL of PBS was added, and the plate was washed once and resuspended in a final volume of 150 µL. The plates were analyzed by using a Cytoflex flow cytometer (Beckman Coulter). The data acquired from the flow cytometer experiments described above were processed on Flowjo software for gating strategy. The positive gate for the live GFP-expressing bacteria was set by gating for granularity (SSC) and GFP fluorescence (FITC) (Supp. Fig 2A). Antibody reactivity was determined by gating on percentage bound bacteria positive for APC-A (Supp. Fig 2A). The data were analyzed in GraphPad Prism, and the percentage of bacteria positive for antibody was plotted on the Y-axis with antibody concentration on the X-axis. A non-linear regression analysis was performed, with Bmax constraint set to 100% while Hill-slope and KD were unconstrained.

### Phagocytosis assay

After heat killing and staining the bacteria as described above, the samples were sonicated for 4 minutes to remove big clumps of aggregated bacteria. The bacteria were diluted 1:500, and 250 µL were added to a 96-well plate and run on Cytoflex flow cytometer (Beckman Coulter) to quantify the concentration as events/µL. In addition, to control the pH responsiveness of the CypHer5e staining, 2 µL of sodium acetate (3M, pH 5.0) was added to the wells to observe a right shift in the APC channel, indicating successful staining. Serial dilutions of bacteria were done with MOP (bacteria per phagocyte) as the variable, and each well was opsonized with 15 µg/mL of anti-M antibodies or Fc-control for 45 minutes at 37 °C gently shaking. 100 µL was the end volume before the addition of the cells. While the plate was opsonizing, the THP-1 cells were collected and washed once to remove RPMI and exchanged to sodium media. THP-1 cells, numbering 1 × 105 cells, were added at a concentration of 2 × 106 per mL after being on ice for 5 minutes. The cells were left phagocytosing for 30 minutes at 37°C while gently shaking. The plate was put on ice for 15 minutes to stop further phagocytosis. The plate was analyzed in the Cytoflex flow cytometer (Beckman Coulter) with 488nm and 638nm lasers and filters 525/40 and 660/10 APC. For bacteria, the threshold was set to FSCH 2000, SSC-H 2000, and for THP-1 cells, FSC-H 70000. The gain was set to 20 for FITC and 265 for APC. The acquisition was set to capture 5000 THP-1 cells with medium velocity (30 µL/min). While running, the 96-well plate was kept on an ice-cold insert. THP-1 cells were gated first by size (FSC) and granularity (SSC) and then for single cells by SSC-A and SSC-H. By using untreated THP-1 cells, we set a gate for non-associated THP-1 cells, associated with bacteria and associated with bacteria and internalized by using the FITC and APC signal (Supp. Fig 1B and Supp Fig 5B). The data acquired from the flow cytometer experiments described above were processed on Flowjo software for gating strategy and later analyzed on Graphpad Prism. The MOP50 analysis was calculated by using the PAN-assay model as described in Prism (20), which in short, is a non-linear regression model where EC50 of MOP is calculated based on the persistent association (association) as an outcome metric with a top value set to 100% and bottom value constrained to 0%. Finally, the phagocytosis score was calculated by multiplying the percentage of bacteria-positive THP-1 cells (associating) with the bacterial signal (MFI, Oregon green) for the whole population of THP-1 cells.

### Animal model

All animal use and procedures were approved by the local Malmö/Lund Institutional Animal Care and Use Committee, ethical permit number 03681-2019. Nine-week-old female C57BL/6J mice (Scanbur/ Charles River Laboratories) were used. Monoclonal antibodies (0.4 mg/mouse) were administered intraperitoneally 6 h pre-infection. The pretreatment groups were coded, and the experimenters were blinded to which group had which intervention, and unmasking was done after the data had been analyzed and compiled. S. pyogenes AP1 was grown to an exponential phase in Todd-Hewitt broth (37°C, 5% CO2). Bacteria were washed and resuspended in sterile PBS. 2.5 x105 CFU of bacteria were injected subcutaneously on the right flank, leading to a local infection that progressed to systemic infection within 24 h. Mice were sacrificed 24 h post-infection, and organs (blood, livers, spleens, and kidneys) were harvested to determine the degree of bacterial dissemination. Cytokines in plasma were quantified using a cytometric bead assay (CBA mouse inflammation kit, BD biosciences) according to manufacturer instructions. The blood from the mice was analyzed by Western blots to control for human IgG integrity at the end of the experiment. We found that most IgG remained intact, and thus appeared unaffected by potential hydrolysis by bacterial enzymes or other potential degradation.

### Structural models

The Fc and Fab domains of IgG1 and IgG3 were de novo modeled separately, using AlphaFold2 (45-46), considering the homo-oligomer state of 1:1. The multiple sequence alignments were generated by MMseqs2 (47). The output models were then relaxed using Rosetta relax protocol (48), where the side chains and disulfide bridges were adjusted. For IgG3, as the hinge region is long and contains multiple disulfide bridges, it was modeled separately using AlphaFold2 and relaxed by Rosetta relax protocol. The loops connecting the hinge region of both IgGs to Fc and Fab domains were then re-modeled and characterized using the DaReUS-Loop web server (49-50). Finally, the full-length structure of both IgGs was relaxed, and all disulfide bridges (specifically in the hinge region) were adjusted using Rosetta relax protocol. The M1- IgG models were generated using a targeted cross-linking mass spectrometry (TX-MS) approach (51). The cross-link constraints (XLs) were derived from a set of experiments generated in our previous study (11), and the threshold of 32 Å was applied to map XLs on the structure. Accordingly, two epitopes of the M1 protein in the B3 and C1 domains interact with the Fab domains of IgGs. The M1-Fab interactions for both IgGs were also separately characterized and re-adjusted using AlphaFold before relaxing the final structure using Rosetta relax protocol.

### Molecular dynamics simulations

We performed three replicates of 1 µs MD simulations starting from the models obtained for M1-IgG1, M1-lgG3, and M1-IgGh systems. This resulted in a total of 9 µs MD simulations. In one replicate of the M1-IgGh system, we observed large conformational changes in the IgGh with respect to the M1. In this replicate, the Fab domains were detached, resulting in a global shift of the antibody and, finally, attachment of the Fc domain to the M1. Therefore, this replicate was discarded from the analysis. The 3D coordinates of the two models generated for M1-IgG1 and M1-IgG3, described in the previous section, were retrieved. Both IgG1 and IgG3 have 6 chains (A-F); four disulfide bonds are present on the Fc domain, and four on each Fab domain. However, the IgG1 has a shorter hinge region of 14 residues (E222-P236) with 2 disulfide bonds, while the hinge region of the IgG3 is composed of 61 residues (E222-P283) and 11 disulfide bonds. The M1 protein has 2 chains (M, N), and for the MD simulations, we considered its central region (residues A149-L291 on chains M and N). MD simulations were carried out with the GROMACS 2020.6 (52) using the CHARMM36m force field parameter set (48): (i) Na+ and Cl- counter-ions were added to reproduce physiological salt concentration (150 mM solution of sodium chloride), (ii) the solute was hydrated with a triclinic box of explicit TIP3P water molecules with a buffering distance of up to 12 Å, and (iii) the environment of the histidine was checked using MolProbity (53). First, we performed 5000 steps of minimization using the steepest descent method keeping only protein backbone atoms fixed to allow protein side chains to relax. After that, the system was equilibrated for 300 ps at constant volume (NVT) and for a further 1 ns using a Langevin piston (NPT)(54) to maintain the pressure while the restraints were gradually released. For every system, three replicates of 1 µs, with different initial velocities, were performed in the NPT ensemble. We applied positional restraints with the force constant of 10 kcal/mol/Å2 on the heavy atoms of the M domain during the production runs to avoid drastic rearrangements. The temperature was kept at 310 K and pressure at 1 bar using the Parrinello-Rahman barostat with an integration time step of 2.0 fs. The Particle Mesh Ewald method (PME) (55) was employed to treat long-range electrostatics, and the coordinates of the system were written every 100 ps. Typically, the M1-lgG1 and M1-lgG3 systems are composed of ~481,152 and ~1,230,941 atoms in a triclinic water box with a volume of ~4,925 and ~12,567 nm3, respectively. The root mean square deviations (RMSD) of the studied complexes from the equilibrated structure were measured on the C$ atoms along simulation time for all the replicates (Fig. 2). All systems were fully relaxed after 100 ns. Then, we calculated the residue root mean square fluctuations (RMSF) over the last 900 ns of every simulation (Fig. 2) with respect to the average conformation and over the Cα atoms.

**Hydrogen bonds and salt bridges:** The hydrogen bonds (H-bonds) were detected using the HBPLUS algorithm (56). H-bonds are detected between donor (D) and acceptor (A) atoms that satisfy the following geometric criteria: (i) maximum distances of 3.9 Å for D-A and 2.5 Å for H-A, (ii) minimum value of 90° for D-H-A, H-AAA and DA-AA angles, where AA is the acceptor antecedent. For a given H-bond between residues i and j, interaction strength is computed as the percentage of conformations in which the H-bond is formed between any atoms of the same pair of residues (i and j). Moreover, the salt bridge plugin of the VMD was used to detect all salt bridges at the interface between the Fab domains and the M1 protein (57). The stability of salt bridges was recorded as the percentage of conformations in which the distance between the center of mass of the oxygens in the acidic side chain and the center of mass of the nitrogens in the basic side chain is within the cut-off distance of 3.5 Å.

**Angles:** We measured the angle formed between 1) the Fc domain and the M1 protein, 2) Fc and Fab domains, and 3) Fab1 and Fab2 domains. The vectors used to calculate each angle are defined here: ● between the Fc domain and the M1 protein: i) the center of mass of the Fc domain and center of mass of the M 1 protein, ii) the center of mass and the Nterminal region of the M 1 protein. ● between the Fc domain and Fab domains: i) the center of mass of the Fc domain and center of mass of the hinge region, ii) the center of mass of the Fab domain and center of mass of the hinge region ● between the Fab1 and Fab2 domains: the center of mass of the hinge region and center of mass of each Fab domain ● Fab2 domain: the center of mass of the hinge region and center of mass of the Fab2 The angles were then defined based on the lines that best fit these segments. The results are reported in two scenarios: for all trajectories (Supp. Fig 3 A-C) and in dualFab binding conformations where the distance between residues at the interface of Fab1 and the M1 protein is less than 30 Å (Supp. Fig 3 D-F). The second scenario resulted in 20,074 angles for M1-lgG1, 10,871 angles for M1-IgG3, and 11,306 angles for M1-IgGh simulations.

### Clustering analysis

To characterize representative conformations, we performed a cluster analysis of the MD trajectories using backbone RMSD as the similarity metrics by the GROMOS clustering approach (58) with a cutoff equal to 0.7 nm. The trajectories of replicates were first concatenated, clustering was then performed, and finally, the population of each cluster was calculated. The same analysis was performed only considering the replicates where the dual-Fab binding was observed (replicates 1 and 3 for M1-lgG1, replicate 3 for M1-IgG3, and replicate 1 for IgGh simulations). Clusters with a population of at least 3,000 conformations were colored in shades of green on the plot (Supp. Fig. 4A). The results are reported for all MD trajectories of M1-lgG1, M1-lgG3 and IgGh systems (30000, 30000 and 20000 conformations, respectively) and only for dual-Fab binding conformations (20000, 10000 and 10000 conformations, respectively). The three highly populated clusters of IgG1 consist of 9071, 4852, and 3239 conformations. And the top three highly populated clusters of IgG3 consist of 5363, 4195, and 4031 conformations. The representative conformations of the highly populated clusters are shown when dual-Fab binding was observed (Supp. Fig. 4BE).

### Crystallization data analysis

The Ab25 Fab sample was concentrated to 6.75 mg/mL in the buffer of 20 mM HEPES(2-[4-(2-hydroxyethyl)piperazin-1-yl]ethane-1-sulfonic acid), 150 mM NaCl, pH 7.0. A crystallization experiment set up against commercially available screens was performed with a Mosquito crystallization robot (SPT Labtech) using sitting drops with a total volume of 300 nL and different ratios between the protein sample and reservoir solution. Optimization of initial hits gave crystals appearing within a week in 27.50% (w/v) PEG8000, 0.1 M CHES pH 8.5. After growing to full size (50x100x250 µm), the crystals were harvested, cryoprotected in reservoir solution supplemented with 20 % (v/v) glycerol, and flash frozen in liquid nitrogen. Testing for diffraction and data collection was performed at the BioMAX beamline at the MAX IV Laboratory (Lund, Sweden). Diffraction data were collected by fine-slicing with an oscillation range of 0.1°, 360° total. The data were auto-processed with the software EDNA (59) to a resolution of 1.88 Å, and the structure was solved by molecular replacement with PHASER of the PHENIX software suite (60), using PDB entry 6JC2.pdb as a search model for both the heavy and light chain. Electron and difference density maps were manually inspected, and the model was improved using Coot (61) and several rounds of refinement with PHENIX (62). The calculation of Rfree used 5.04 % of the data. Statistics over data and refinement can be seen in Supplementary Table 1. The crystallographic data have been deposited to the Protein Data Bank (PDB) with the accession ID: 8C67. Statistical analysis Statistical analysis was performed with Kruskal-Wallis multiple comparisons test with Dunn's correction test with GraphPad Prism.

### Example 1: IgG3 exhibits potent phagocytosis efficacy despite reduced binding to antigen.

Previously, we generated a human IgG1 antibody, Ab25, against the M protein of Streptococcus pyogenes which was opsonic in vitro and protective in vivo (11). This antibody was generated using a human B cell from a convalescent donor. The original subclass is not known. Using PCR and homologous recombination, we exchanged the subclass-specific domains of the heavy chain, generating three new plasmids encoding the remaining subclasses IgG2, IgG3, and IgG4 (Fig. 1A). The hinge length varies, with IgG1 having a 15 aa flexible hinge, IgG2 having a 12 aa rigid hinge (due to more disulfide bridges), and IgG4 having a 12 aa flexible hinge. IgG3 has about four times longer hinge region, distributed across 13 different allotypes with up to 62 aa hinge length (1), and for this study, we used allotype IGHG3*11 with a 62 aa hinge. In previous work, we attempted to measure the affinity of Ab25 IgG1 to both purified and recombinant M protein by using surface plasmon resonance (SPR). These attempts were unsuccessful. This is most likely due to the M protein's conformational stability being highly dependent on temperature, dimerization, and being attached to its native bacterial surface (19). Instead, we measured the direct functional affinity (likely equivalent to avidity) of antibodies to M protein on live bacteria (Supp Fig. 1A) (11). We calculated the dissociation constant (KD) for all Ab25 subclasses using a nonlinear regression analysis. This analysis revealed different affinity profiles across the subclasses (Fig. 1B). IgG1, IgG2, and IgG4 showed similar affinities (lgG1 KD = 2.2; IgG2 KD = 1.8; IgG4 KD 4.7 nM-1, respectively). IgG3 showed more than a 14-fold decrease in affinity compared to IgG1 (lgG3 KD = 28.3 nM-1). Our results indicate that subclass-switching Ab25 IgG1 to IgG3 leads to reduced binding to the M protein, while for IgG2 and IgG4, binding affinity is retained. Having established the binding properties of the Ab25 IgG subclass variants, we investigated their functional output in terms of Fc-mediated phagocytosis. We used a previously established opsonophagocytosis assay for S. pyogenes with heat-killed SF370 bacteria and human phagocytes of the monocytic THP-1 cell line (11, 20). The opsonic ability of the subclasses was assessed by varying the ratio of prey to phagocyte (multiplicity of prey, MOP). We calculated the number of bacteria relative to phagocytes needed to elicit a 50% association (MOP50) (20). A lower MOP50 value means it is more efficient at mediating bacteria-monocyte interaction. The MOP50 analysis showed that IgG3 exhibited a potent opsonic phenotype (Fig. 1C). There was a 6-fold increase in opsonic ability (MOP50 = 12) compared to the original Ab25 IgG1 (MOP50 = 68) and a 12-16-fold increase compared to IgG2 (MOP50 = 145) and IgG4 (MOP50 = 192) (Fig. 1D-E). To put these values into context, the negative Fc-control exhibited a 30-fold lower efficacy compared to IgG3 (MOP50 = 367). We analyzed the percentage of phagocytes with internalized bacteria and saw a similar outcome as the MOP50 analysis with the following order IgG3>IgG1>IgG2>IgG4. (Fig. 1F). Additionally, when considering the bacterial signal, a quantity metric, we assessed the relative quantity of bacteria phagocytosed (Supp Fig. 1B-C). This analysis shows that IgG3 promotes the most efficient phagocyte-bacteria interaction (Fig. 1G). Finally, we calculated a phagocytosis score based on the combined phagocyte association and bacterial signal (MFI) of the THP-1 population. This analysis shows that IgG3 is more potent than the other subclasses, with a 7-fold higher score than IgG1 at MOP 200 (41600 vs. 6900, Fig.1H-I), 26-fold higher than both IgG2 and IgG4 (1600 respectively) and 130-fold higher than control (300). The four assessed metrics (MOP50, internalization, bacterial signal, and phagocytosis score) showed that Ab25 IgG3, despite its reduced reactivity to M1, is a more potent opsonizing antibody than the other subclasses.

### Example 2: The IgG3 hinge provides broad Fc spatial mobility spectra and flexibility relative to M protein

Our in vitro findings of increased opsonic ability of IgG3 align with other recent findings with increased Fc-effector functions of this subclass (21-23). However, we observed a discrepancy with lower binding yet stronger functional output. We hypothesized that this increased functionality is due to the extended hinge region of IgG3. To investigate this, we engineered a hybrid mAb containing the backbone of IgG1 but with the hinge of IgG3 (IgGh). Affinity measurements with directly labeled IgG1 and IgGh antibodies showed that the antigen binding appeared to be unaffected by this modification (Supp. Fig. 2B). To assess if the hinge of IgG3 grants increased Fc flexibility, we performed three replicates of 1 µs molecular dynamics (MD) simulations for IgG1, IgG3, and the IgGh (see Methods section for the details of simulations and Supplemental Movies 1- 9). We determined a crystal structure of the Ab25 Fab to enhance the accuracy of the simulations (Supp. Table 1, PDB ID: 8C67). We have previously found that the Ab25 binds with both Fabs to two different epitopes on the M protein, so-called dual-Fab cis binding (11). The data obtained by protein cross-linking mass spectrometry was supported by experimental validation showing that the single Fabs of Ab25 IgG1 could barely bind to M protein compared to F(ab')2 fragments (11). Our current MD analysis of the Fab-M1 interactions thus focused on this dual-Fab cis interaction. To evaluate the stability of the generated trajectories, we measured the root mean square deviations (RMSD) of different domains (M1 protein, Fc, Fab1, and Fab2) with respect to the equilibrated starting structure (Fig. 2 A-D). The three systems of IgG1, IgG3, and IgGh represent similar patterns of RMSD within the Fc domain (average values of 5.34±0.88, 4.35±0.91 and 5.28±0.84 Å, respectively), and Fab2 region (average RMSD of 4.89±0.89, 5.16±0.77 and 4.43±0.8 Å, respectively, Supp. Table 2). The Fab1 domain of the IgG1 system showed lower RMSD values (average of 3.72±0.64) with respect to the Fab1 of both the IgG3 and IgGh (average values of 5.76±1.06 and 6.49±1.48 Å, respectively). We also measured the root mean square fluctuations (RMSF) of every residue within different domains with respect to the average conformation (Fig. 2 E-H). Both IgG3 and IgGh exhibited larger fluctuations of Fab1 (average RMSF of 2.18±1.16 and 2.32±1.33 Å, respectively) compared to IgG1 (average RMSF of 1.92±0.86 Å), hinting at weaker binding to M1 for this Fab (Supp. Table 2). More interestingly, IgGh displayed larger fluctuations and flexibility in its Fc region (average RMSF of 2.88±1.68 Å) compared to both IgG1 and IgG3 (average RMSF of 1.64±1.01 and 1.63±0.96 Å, respectively). Surprisingly IgGh showed smaller fluctuations within Fab2 (average RMSF of 1.62±0.65 Å) compared to both IgG1 and IgG3 (average RMSF of 2.55±0.94 and 2.57±1.24 Å, respectively). We proceeded to investigate the dynamics of the Fc of the three mAbs relative to M1. We calculated the displacement between the center of mass of the Fc domain of all mAbs and the M1 protein over the time course of the simulations (Fig. 3A). The results showed larger displacement for both IgG3 and IgGh with average values of 111.01±24.50 and 167.13±17.60 Å, respectively, compared to IgG1 (94.10±6.77 Å Supp. Table 2). The larger standard deviations for both IgG3 and IgGh systems suggest the higher flexibility of the Fc domain in those two systems with respect to the M1 protein. To further analyze the flexibility of the Fc domain in 3D space, we recorded the position of the center of mass of the Fc domain from all three mAbs in every conformation of the MD simulations, with the center of mass of the M1 protein maintained at the origin (Fig. 3B). Individual representations for the three systems are depicted in Figure 3C-3E. This analysis showed that the Fc domain of both the IgG3 and IgGh systems spans a larger 3D volume compared to the Fc domain of IgG1. Moreover, the traces of the Fc domain from IgG3 showed larger degrees of bending that bring the Fc domain closer to the M1 protein. However, in the case of IgGh, while the Fc domain remained flexible, it kept a certain distance from the M1 protein and explored the space differently. Thus, the hinge of IgG3 leads to both larger degrees of freedom and more flexibility, while the IgG1 hinge confers spatial constraints.

Furthermore, we measured three sets of angles: i) between the Fc domain of the IgGs and the M1 protein, ii) between the Fc and Fab domains of the IgGs and iii) between the two Fab domains of the IgGs (Fig. 3F). These angles were recorded from all MD conformations (Supp. Fig. 3 A-C); however, only the values where both Fab domains are in the proximity (<30 Å) of the M1 protein are of interest to this study due to the dual-fab binding phenotype of Ab25. Thus, focus was on the conformations where dual-Fab binding occurred (Supp. Fig. 3 D-F).

The Fab1-Fab2 angles showed that the antibodies were tilted in different ways but with no substantial variation in the three systems during the simulations (Supp. Fig. 3F). The changes of Fab1-Fab2 angles for IgG1 are in the range of 86° and 139° (with an average value of 111°±15°), for IgG3 between 37° and 80° (with an average value of 62°±8°), and for IgGh in the range of 19° and 57° (with the average value of 38°±7°, Supp. Table 2). For the Fc-Fab angles (Supp. Fig. 3E, Supp. Table 2), we observed larger distributions of angles for IgG1, IgG3, and IgGh within the ranges of 13° - 114°, 1° - 117°, and 0° - 77°, respectively. The average values of the Fc-Fab angles for the three systems were 66°±22°, 72°±31°, and 31°±14°, respectively. The angle span of the Fc-Fab for IgGh (77°) is roughly two-thirds of the Fc-Fab angle span in IgG1 (101°) and IgG3 (116°) systems. Considering the Fc-M1 angles (Supp. Fig. 2D, Fig. 3F), the following spans were observed: 69° - 108° for IgG1, 34° - 113° for IgG3 and 66° - 132° for IgGh, with the average values of 92° ± 9°, 86° ± 18° and 93° ± 14°, respectively. Thus, IgG3s Fc exhibits twice as large an angle span (79° vs. 39°) and IgGh 1.7-fold (66° vs. 39°) compared to IgG1s Fc relative to the M1 protein (Supp. Table 2). Models for Fc receptor activation suggest that the accessibility of Fc domains is important to increase the avidity of the interactions (24-25). Therefore, we further analyzed the most prevalent configurations (clusters) regarding the orientation of the Fc relative to the M1 protein for the respective antibodies (Supp Fig. 4). Cluster analysis of the MD trajectories revealed that dual-Fab binding occurred in two out of the three most prevalent clusters of the M1-IgG1 system. At the same time, the binding of only Fab2 was observed in the three most significant clusters of the M1-IgG3 system (Supp. Fig. 4A). The results showed that, on average, the Fc domain of IgG1 is in a perpendicular-like position with respect to the M1 protein in the three most representative conformations (Supp. Fig. 4B-D). The most prevalent configuration for IgG3 Fc is when it bends from the hinge region and fluctuates in parallel to the M1 protein away from the bacterial surface (Supp. Fig. 4E, cluster 1 for the IgG3-M1 system Supp. Fig. 4A). For IgGh, we detected many clusters with small populations of conformations. This suggests large conformational changes and high flexibility of the IgG hybrid system during the simulations (Supp Fig. 4A). These results showed that the IgG3 hinge region increased the movement span. Taken together, the IgG3 hinge region provided IgG3 and IgGh with broad spectra of Fc mobility and flexibility relative to the M1 in 3D space, where IgGh gained IgG3's increased flexibility through the exchange of IgG1's hinge with that of IgG3's.

### Example 3: The constant domain influences the interaction network between the IgG Fabs and M1 at an atomic level

For the simulations analyzed across all three IgG-M1 systems the binding of Fab2 to the M1 was stable for all three antibodies, while the Fab1 showed a transient behavior and was most stable for IgG1 (Fig. 2C-D, Fig. 2G-H). One replicate of IgGh became dislodged from M protein during the simulation and was excluded from further analysis. To better understand the binding stability of both Fabs across all MD trajectories, we extracted the network of hydrogen bonds (Hbond) and salt bridges at the interface between the Fab domain of IgGs and the M1 protein (Fig. 4). This analysis revealed two stable salt bridges (E44-K58; E46-K65) formed between the VH domain in Fab2 of both IgG1 and IgG3 and the M1 protein (Fig. 4A-B). For the IgGh replicates, two salt bridges (E46-K56; E46-K58) occurred, but only one salt bridge per replicate. These salt bridges provide insight into the stability of Fab2 seen during the simulations. Interestingly, although IgGh lacks the presence of a salt bridge in one of the two simulations (in one simulation, the E46-K56 is absent, and in the other, it is E46-K58), the analysis also revealed a new salt bridge between Fab1 and M1 (E76-K127). IgG3 and IgG1 did not share this interaction, and neither of these antibodies formed a salt bridge between their Fab1 and M1. Moreover, we recorded a set of eight H-bonds for the Fab1 domain of IgG1, whereas only two Hbonds were observed for the Fab1 domain of IgG3 and IgGh (Fig. 4C-E). These hydrogen bonds that IgGh and IgG3 form are not identical and are formed by different amino acids. In addition, these amino acids in Fab1 of IgG3 and IgGh bind to different residues on the M1 protein, which do not interact with Fab1 of IgG1. The reduction of hydrogen bond interactions in Fab1 between IgG3-M1 and IgGh-M1 could explain the less stable binding of Fab1 during the simulations. Furthermore, there are notable differences between the three IgG-M1 systems in the Fab2 hydrogen bond interaction network. IgG3 and IgGh Fab2 both form six and seven Hbonds, respectively, with the M1 epitope, while IgG1 Fab2 forms only two (Fig. 4C-E). It is important to note that the Fab2-M1 interactions for all three antibodies are very stable in the simulation (Fig. 2C-D, Fig. 2G-H). IgG1s Fab2, despite the reduced amount of Hbonds, exhibits stable binding to M1, most likely due to the presence of two salt bridges mentioned prior (E44-K58; E46-K65). The two Hbonds of IgG1 in the Fab2 (E44-K58; E46-K65) are both shared by IgG3, and IgGh shares one (E46-K65). One of the remaining Hbonds is shared between the IgGh and IgG3 Fab2 (R46-R56). Different amino acids form the remaining Hbonds. Taken together, the analysis of the network of interactions reveals differences in both Fabs across all three IgG-M1 systems. The differences between IgG3 and IgG1 in Fab1 could explain the reduced binding of IgG3 to M1 measured previously (Fig. 1B). The Fab2 interactions with M1 appears more stable due to the salt bridges in all IgG systems. In terms of hydrogen bond formation, the IgGh network of interactions seems to be similar to IgG3. However, an exception is that IgGh Fab1 forms a new salt bridge with M1. Thus, despite the reduction of Hbonds in IgGh Fab1-M1, IgGh binding to M1 is compensated by the presence of this novel salt bridge (E76-K127) which could explain its IgG1-like reactivity to M1. Nonetheless, our analysis revealed that the network of atomic interactions between the antigen and antibody Fabs is altered when exchanging parts of (IgG3 hinge region) or the entire constant domain.

### Example 4: IgGh retains the high affinity of IgG1 and gains superior opsonic function

The MD simulations highlighted the impact of the IgG3 hinge on increased spatial Fc movement and relative flexibility to the M1 protein. To investigate how the Fc mobility and flexibility influence antibody function, we compared the IgGh opsonic function to that of its parent subclasses (Fig. 5A). First, we analyzed IgGh's ability to promote phagocyte-bacteria association by calculating the MOP50. In these sets of experiments, the differences in MOP50 between IgG3 and IgG1 were 3-fold (IgG3=21, and IgG1=62) (Fig. 5B-C). Adding the IgG3 hinge improved the opsonic efficacy for IgGh 2.5-fold over IgG1 (MOP50= 25, Fig. 5B-C). All three mAbs had a much higher association than the negative control (MOP50= 188), also reflected in the individual MOP50 analysis (Fig. 5D). We further assessed the importance of hinge flexibility by looking at the proportion of THP-1 cells with internalized bacteria and the bacterial fluorescence. The IgGh outperformed both parent subclasses in internalization with more phagocytes internalizing bacteria (Fig. 5E). We also analyzed the quantity of bacteria phagocytosed, which was the highest with IgGh as the opsonin (Fig. 5F). Finally, we calculated a phagocytosis score (percentage of associating cells of the whole population multiplied by the bacterial signal of the whole cell population), and the results from this analysis showed that IgGh exceeds both the potent lgG3 and the original IgG1 (Fig. 5G-H). At MOP 200, IgGh has a 2.5-fold higher phagocytosis score than IgG3 (50.400 vs 20.600), 4-fold over IgG1 (12900), and 35-fold over control (1400). Taken together, these results strengthen the hypothesis that the spatial Fc mobility and flexibility enabled by IgG3's hinge is a crucial factor for potentiating Fcmediated phagocytosis. Our data also showed that IgGh is a more potent opsonin than IgG3 when all four metrics are considered together.

### Example 5: Increased hinge flexibility of IgG1 confers antibody protection against systemic infection in mice

To study the biological relevance of increased Fc flexibility, we proceeded with in vivo experiments. We used an animal model of invasive infection and passive immunization where C57BL/6J female mice were prophylactically pretreated 6 hours before infection with IgG1, IgG3, IgGh, or PBS (Fig. 6A). Bacteria were then administered on the flank in a low volume to generate a localized tissue infection, which differs from the previous model used to assess Ab25 IgG1, where the mice were injected with higher volumes in the scruff to generate a diffuse tissue infection (11). We assessed the protective effect of the antibodies by quantifying bacterial dissemination to distant organs (spleen, liver, and kidneys) and systemic cytokine mobilization in blood. Interestingly, in this model, both IgG3 Ab25 and IgG1 Ab25 treatment of mice reduced bacterial spread to distant organs compared to the PBS control but did not completely prevent systemic dissemination. Distinct organotypic patterns of dissemination were observed (Fig. 6B-D). IgG3-treated mice did not reduce bacterial load in the spleen compared to PBS (median 9800 CFU/g vs. 7200, Fig. 6B), while mice treated with IgG1 showed a reduced spread to the spleen (median 1400 CFU/g Fig. 6B). In the liver, IgG3 reduced the bacterial load to a greater extent than IgG1 (median 3200 CFU/g vs. 10800) and compared to the PBS group (median 55600 CFU/g, Fig. 6C). In the kidneys, there was a decrease in bacteria for mice in both IgG1 and IgG3-treated groups (median 1000 CFU/g and 1200 respectively) compared to the PBS-treated mice (median 3200 CFU/g, Fig. 6D). In contrast, IgGh did not have any detectable bacterial presence for a majority of the cohort (undetectable bacteria were labeled as 14 CFU/g in the graph, IgGh had median 14 CFU/g for all organs, Fig. 6B-D). More importantly, the control group only had one mouse (10%) in the cohort that was bacteria-free in all three organs (Fig. 6E-F). For IgG1, 30% of all mice were bacteria-free in the spleen (Fig. 6E), 20% in the liver (Fig. 6F), and 40% in the kidneys (Fig. 6G). This was comparable to the IgG3 response, with 20% for the spleen, 30% for the liver, and 20% for the kidneys. IgGh stands out in this analysis, with 70% of the cohort not having bacteria in the spleen, 80% in the liver, and 60% in the kidneys (Fig. 6E-G). We analyzed cytokine expression in the blood of each respective mouse in all groups. Large individual variation in each group was observed for MCP-1, TNF-alpha, and IL-6. All three mAb-treated groups had equal MCP-1 compared to the control group (Fig. 6H) and similar TNF-alpha response (Fig. 6I). For IL-6, there was a clear distinction between IgG3-treated groups and the other groups (Fig. 6J). The almost complete prevention of bacterial dissemination to distant organs combined shows that the IgGh is the most protective antibody. Although IgG3 has extensive Fc-mobility equal to IgGh, it did not provide similar levels of protection, possibly explained by its reduced half-life, lower affinity, or different Fc-receptor affinity towards mouse Fc-gamma receptors. However, comparing IgG1 and IgGh does not suffer from these confounders. It highlights that Fc spatial flexibility and mobility can be determining factors for complete immune protection against systemic streptococcal infection.

### List of reference.

1. Vidarsson G, Dekkers G, Rispens T. IgG subclasses and allotypes: From structure to effector functions. Frontiers in Immunology. 2014;5(OCT):1-17.
2.Roco JA. et al. Class-Switch Recombination Occurs Infrequently in Germinal Centers. Immunity.2019;51(2):337-350.e7.
3.Oi, V. et al. Correlation between segmental flexibility and effector function of antibodies. Nature 307, 136-140 (1984).
4. Torres M, Casadevall A. The immunoglobulin constant region contributes to affinity and specificity. Trends in Immunology. 2008;29(2):91-7.
5. Janda A, Bowen A, Greenspan NS, Casadevall A. Ig constant region effects on variable region structure and function. Frontiers in Microbiology. 2016;7(FEB):1-10.
6. Janda A, Eryilmaz E, Nakouzi A, Cowburn D, Casadevall A. Variable region identical immunoglobulins differing in isotype express different paratopes. Journal of Biological Chemistry. 2012;287(42):35409-17.
7. Hovenden M. et al. IgG Subclass and Heavy Chain Domains Contribute to Binding and Protection by mAbs to the Poly γ-D-glutamic Acid Capsular Antigen of Bacillus anthracis. PLoS Pathogens. 2013;9(4):1-11
8. Roux KH, Strelets L, Michaelsen TE. Flexibility of human IgG subclasses. J Immunol. 1997;159(7):3372-82.
9.Bindon C. I., Hale G., Brüggemann M., Waldmann H., Human monoclonal IgG isotypes differ in complement activating function at the level of C4 as well as C1q. J. Exp. Med. 168, 127-142 (1988).
10. Bruhns P. et al. Specificity and affinity of human Fcy receptors and their polymorphic variants for human IgG subclasses. Blood. 2009;113(16):3716-25.
11. Bahnan W. et al. (2022). A human monoclonal antibody bivalently binding two different epitopes in streptococcal M protein protects against infection. EMBO Mol. Med. 14, 1-21.
12. Mcnamara C. et al. Streptococcus M1 Are Required for Virulence.Science. 2008;319(5868):1405-8.
13.Nordenfelt P. et al. Antibody orientation at bacterial surfaces is related to invasive infection.J. Exp. Med. 2012; 209(13):2367-81.
14.Khakzad H. et al. Structural determination of Streptococcus pyogenes M1 protein interactions with human immunoglobulin G using integrative structural biology. PLoS Computational Biology. 2021;17(1):1-19.
15. Wrighton S, Ahnlide Kumra V, Andre O, Bahnan W, Nordenfelt P. Group A streptococci induce stronger M protein-fibronectin interaction when specific human antibodies are bound. Frontiers in microbiology. 2023; 14.
16.Carlsson F, Sandin C, Lindahl G. Human fibrinogen bound to Streptococcus pyogenes M protein inhibits complement deposition via the classical pathway. Molecular Microbiology. 2005;56(1):28-39.
17. Dale JB, Walker MJ. Update on group A streptococcal vaccine development. Curr Opin Infect Dis. 2020 Jun;33(3):244-250.
18. Happonen, L. et al. A quantitative Streptococcus pyogenes-human protein-protein interaction map reveals localization of opsonizing antibodies. Nat Commun 10, 2727 (2019). https://doi.org/10.1038/s41467-019-10583-5
19.Nilson BH. et al. Structure and stability of protein H and the M1 protein from Streptococcus pyogenes. Implications for other surface proteins of gram-positive bacteria. Biochemistry. 1995 Oct 17;34(41):13688-98.
20. de Neergaard T, Sundwall M, Wrighton S, Nordenfelt P. High-Sensitivity Assessment of Phagocytosis by Persistent Association-Based Normalization. J Immunol 2021;206(1):214-24.
21.Varshney AK. et al. A natural human monoclonal antibody targeting Staphylococcus Protein A protects against Staphylococcus aureus bacteremia. PLoS ONE. 2018;13(1):1-22.
22. Stapleton NM. et al. Competition for FcRn-mediated transport gives rise to short half-life of human IgG3 and offers therapeutic potential. Nat Commun. 2011;2(1).
23.Chu TH. et al. Hinge length contributes to the phagocytic activity of HIV-specific IgG1 and IgG3 antibodies. PLoS Pathogens [Internet]. 2020;16(2):1-25.
24.Woof JM, Burton DR. Human antibody-Fc receptor interactions illuminated by crystal structures. Nature Reviews Immunology. 2004;4(2):89-99.
25.Radaev S, Sun P. Recognition of immunoglobulins by Fcy receptors. Molecular Immunology. 2002;38(14):1073-8
26. Lu RM. et al. Development of therapeutic antibodies for the treatment of diseases. J Biomed Sci. 2020, 27(1)
27. Kaplon H, Crescioli S, Chenoweth A, Visweswaraiah J & Reichert JM. Antibodies to watch in 2023. mAbs. 2023, 15:1
28. Hamitt LL. et al. Nirsevimab for Prevention of RSV in Healthy Late-Preterm and Term Infants. N. Engl. J. Med. 2022; 386:837-846
29.Brinda Emu et al. Phase 3 Study of Ibalizumab for Multidrug-Resistant HIV-1. N Engl J Med. 2018; 379:645-654
30. Chen P. et al. SARS-CoV-2 Neutralizing Antibody LY-CoV555 in Outpatients with 703 Covid-19. N. Engl. J. Med. 2021, 384, 229-237.
31. Kallolimath S. et al. Highly active engineered IgG3 antibodies against SARS-CoV2. Proc. Natl. Acad. Sci. U. S. A. 2021, 118, 3-4.
32. lzadi A. et al. Subclass-switched anti-spike IgG3 oligoclonal cocktails strongly enhance Fc-mediated opsonization. Proc. Natl. Acad. Sci. U. S. A. 2023, 120 (15).
33. Giuntini S, Reason DC, Granoff DM. Combined roles of human IgG subclass, alternative complement pathway activation, and epitope density in the bactericidal activity of antibodies to meningococcal factor H binding protein. Infection and Immunity. 2012;80(1):187-94.
34.Chu TH, Patz EF, Ackerman ME. Coming together at the hinges: Therapeutic prospects of IgG3. MAbs . 2021;13(1). https://doi.org/10.1080/19420862.2021.1882028
35.Saito S, Namisaki H, Hiraishi K, Takahashi N, lida S. A stable engineered human IgG3 antibody with decreased aggregation during antibody expression and low pH stress. Protein Sci. 2019 28(5):900-909.
36.Foss S. et al. Potent TRIM21 and complement-dependent intracellular antiviral immunity requires the IgG3 hinge. Sci Immunol. 2022;7(70):eabj1640.
37.Woof JM, Burton DR. Human antibody-Fc receptor interactions illuminated by crystal structures. Nature Reviews Immunology. 2004;4(2):89-99.
38. Yu X. et al. Reducing affinity as a strategy to boost immunomodulatory antibody agonism. Nature. 2023 ;614(7948):539-547.
39.Bazin R. et al. Use of hu-IgG-SCID mice to evaluate the in vivo stability of human monoclonal IgG antibodies. J Immunol Methods. 1994, 172(2):209-17.
40. Pierre Bruhns; Properties of mouse and human IgG receptors and their contribution to disease models. Blood 2012; 119 (24): 5640-5649.
41. Dekkers G et al. Affinity of human IgG subclasses to mouse Fc gamma receptors. mAbs. 2017 9(5):767-773.
42. Overdijk MB et al. Crosstalk between human IgG isotypes and murine effector cells. J Immunol 2012; 189:3430-8.
43.Steplewski Z et al. Biological activity of human-mouse IgG1, IgG2, IgG3, and IgG4 chimeric monoclonal antibodies with antitumor specificity. Proc Natl Acad Sci U S A 1988; 85:4852-6
44. Dodev TS et al. A tool kit for rapid cloning and expression of recombinant antibodies Sci Rep. 2014; 4:5885. doi: 10.1038/srep05885. 10.1038/srep05885
45.Mirdita M, Schütze K, Moriwaki Y, Heo L, Ovchinnikov S, Steinegger M. ColabFold - Making protein folding accessible to all. Nature methods. 2022;19:679-682
46. Jumper, J., Evans, R., Pritzel, A. et al. Highly accurate protein structure prediction with AlphaFold. Nature 596, 583-589 (2021). https://doi.org/10.1038/s41586-021-03819-2
47. Steinegger M, Söding J. MMseqs2 enables sensitive protein sequence searching for the analysis of massive data sets. Nature Biotechnology. 2017;35(11):1026-8.
48. Leman JK. et al. Macromolecular modeling and design in Rosetta: recent methods and frameworks. Nature Methods. 2020;17(7):665-80.
49. Karami Y. et al. DaReUS-Loop: a web server to model multiple loops in homology models. Nucleic Acids Research. 2019;47(W1):W423-8.
50. Karami Y, Guyon F, de Vries S, Tufféry P. DaReUS-Loop: accurate loop modeling using fragments from remote or unrelated proteins. Scientific Reports. 2018;8(1):1-12.
51. Hauri S. et al. Rapid determination of quaternary protein structures in complex biological samples. Nat Commun. 2019;10(1):1-10.
52. van der Spoel D et al. GROMACS: Fast, flexible, and free. Journal of Computational Chemistry. 2005;26(16):1701-18.
53. Williams CJ. et al. MolProbity: More and better reference data for improved allatom structure validation. Protein Science. 2018;27(1):293-315.
54. Loncharich RJ, Brooks BR, Pastor RW. Langevin dynamics of peptides: The frictional dependence of isomerization rates of N-acetylalanyl-N'-methylamide. Biopolymers. 1992;32(5).
55. York DM, Darden TA, Pedersen LG. The effect of long-range electrostatic interactions in simulations of macromolecular crystals: A comparison of the Ewald and truncated list methods. The Journal of Chemical Physics. 1993;99(10).
56. York DM, Darden TA, Pedersen LG. The effect of long-range electrostatic interactions in simulations of macromolecular crystals: A comparison of the Ewald and truncated list methods. The Journal of Chemical Physics. 1993;99(10).
57. I.K. McDonald and J.M. Thornton (1994), "Satisfying Hydrogen Bonding Potential in Proteins", JMB 238:777-793
58. Humphrey W, Dalke A, Schulten K. VMD: visual molecular dynamics. J Mol Graph 1996;14:33-8.
59. Daura X, Gademann K, Jaun B, Seebach D, van Gunsteren WF, Mark AE. Peptide folding: When simulation meets experiment. Angewandte Chemie-International Edition. 1999;38(1-2):236-40.
60.EDNA: Incardona, M. F.; Bourenkov, G. P.; Levik, K.; Pieritz, R. A.; Popov, A. N.; Svensson, O. EDNA: A Framework for Plugin-Based Applications Applied to X-Ray Experiment Online Data Analysis. J. Synchrotron Radiat. 2009, 16 (6), 872-879.
61. Adams P.D. et al. PHENIX: A comprehensive Python-based system for macromolecular structure solution. Acta Crystallogr. D. 2010;66:213-221.
62.Emsley P., Lohkamp B., Scott W., Cowtan K. Features and Development of Coot. Acta Cryst D. 2010;66:486-501.

## Claims

1. An engineered antibody comprising an IgG3 hinge and a variable domain having a part binding to the M protein of group A streptococci.

2. The engineered antibody according to claim 1, wherein the Fc region is an IgG1 region.

3. The engineered antibody according to claim 1 or 2, wherein said engineered antibody comprises an IgG1 constant domain engineered with an IgG3 hinge, e.g. SEQ ID NO:3.

4. The engineered antibody according to any of claims 1-3, wherein said IgG3 hinge comprises an amino acid sequence which has at least 90% identity, at least 95% identity, at least 98% identity or 100% identity to the amino acid sequence ELKTPLGDTTHTCPRCPEPSKCDTPPPCPRCPEPSKCDTPPPCPRCPEPSKCDTPPPCPRCP (SEQ ID NO:4) or the amino acid sequence ELKTPLGDTTHTCPRCPEPSKCDTPPPCPRCPEPSKCDTPPPCPRCP (SEQ ID NO:15).

5. The engineered antibody according to any of claims 1-4, wherein said IgG3 hinge comprises an amino acid sequence which has at least 90% identity, at least 95% identity, at least 98% identity or 100% identity to the amino acid sequence X-(Y)n, wherein
X is ELKTPLGDTTHTCPRCP (SEQ ID NO:5),
Y is EPSKCDTPPPCPRCP (SEQ ID NO:6), and
n is an integer in the range from 2 to 8, preferably in the range from 2 to 5, more preferably in the range from 2 to 4.

6. The engineered antibody according to any of claims 1-5, which promotes opsonisation.

7. The engineered antibody according to any of claims 1-6, wherein said engineered antibody has a CDR H3 loop selected from SEQ ID NO:7, SEQ ID NO:8, SEQ ID NO:9, SEQ ID NO:10.

8. The engineered antibody according to any of claims 1-7, wherein said engineered antibody has a CDR L3 loop selected from SEQ ID NO:11, SEQ ID NO:12, SEQ ID NO:13 and SEQ ID NO:14.

9. The engineered antibody according to any of claims 1-8 for use in the treatment of an infection with group A streptococci.

10. A composition comprising at least two different engineered antibodies as defined in any of claims 1-9.

11. A nucleic acid molecule encoding a protein comprising the engineered antibody as defined in any of claims 1-9.

12. A cell comprising the nucleic acid as defined in claim 11, such that the engineered antibody can be produced by said cell.

13. Use of the engineered antibody as defined in any of claims 1-9 or the composition as defined in claim 10, for preventing, treating or alleviating an infection caused by group A streptococci.

14. Use of the engineered antibody as defined in any of claims 1-9 or the composition defined in claim 10, for Fc-mediated phagocytosis of group A streptococci.

15. Use of the composition as defined in claim 10, for activating the complement pathway.

16. A method for preparing an engineered antibody as defined in any of claims 1-9, comprising the steps:
- immunization of a host organism with said group A streptococci or the M protein therefrom,
- isolation of at least one group A streptococci reactive B-cell to isolate an IgG1 antibody binding to said group A streptococci,
- preparing at least one recombinant DNA molecule which encodes a modified version of said IgG1 antibody, said modification comprising the replacing of the IgG1 hinge of said isolated IgG1 antibody with an IgG3 hinge,
- expressing said at least one recombinant DNA molecule in a suitable host cell,
to obtain said engineered antibody.
